# NEW EUROPEAN PATENT SPECIFICATION

(11) **EP 1 603 505 B2**
(45) Date of publication and mention of the opposition decision: **25.02.2015**
(45) Mention of the grant of the patent: 21.12.2011
(21) Application number: 04719169.7
(22) Date of filing: 10.03.2004
(51) Int. Cl.: A61F 13/49, A61F 13/494

(54) **ABSORBENT ARTICLE**
SAUGFÄHIGER ARTIKEL
ARTICLE ABSORBANT

(30) Priority: 14.03.2003 SE 0300715
(43) Date of publication of application: 14.12.2005
(73) Proprietor: SCA Hygiene Products AB, 405 03 Göteborg (SE)
(72) Inventor: CORNELIUSSON, Helena, S-445 34 Bohus (SE)
(74) Representative: Gray, Helen Mary
(86) International application number: PCT/SE2004/000348
(87) International publication number: WO 2004/080359

(56) References cited:
- EP-A1- 0 243 013
- EP-A2- 0 264 952
- EP-A2- 0 750 894
- EP-A2- 0 951 890
- EP-A2- 1 273 281
- EP-B1- 0 433 951
- WO-A1-02/49560
- JP-A- S6 477 609
- US-A- 5 876 390

## Description

### TECHNICAL FIELD

The present invention relates to an absorbent article comprising an upper, liquid-permeable cover sheet, a lower, liquid-impermeable cover sheet, and an absorption body arranged between the cover sheet, said article defining a longitudinal direction, a front portion in the longitudinal direction, a rear portion, and a middle portion arranged between said portions, said article additionally comprising first and second side barriers along the respective longitudinal side, and each side barrier in turn comprising at least one longitudinal elastic element, said first and second side barriers, viewed from above, defining a shape which narrows in the direction towards said front portion so that the distance, in the transverse direction of the article, between said elastic elements is greater in said rear portion than in said front portion.

### BACKGROUND ART

In connection with absorbent articles such as diapers, incontinence protectors for adults, and sanitary towels, there has long been a general need for materials and structures which are able to take up, distribute and absorb bodily excretions in a rapid and effective manner. Today's absorbent articles generally have good absorbency with a low risk of leakage and a high degree of comfort for the person wearing the absorbent article.

The requirement for rapid and effective absorbency is important not least in the case of diapers for very small infants and also for premature babies (i.e. babies born before pregnancy has reached its full term). In this connection, it should be noted that infants and premature babies produce excrement which is loose and runny in consistency. With today's diapers for infants, there is a risk of this loose excrement leaking at the sides in the crotch area and out towards the user's legs, and also along the back of the person wearing the diaper. Such leakage may entail a risk of, for example, soiling of clothes and bed linen. In general, it may be stated that, in connection with diapers for infants, ever greater demands are being placed on the ability to take up excreted material.

According to the prior art, a diaper for infants is normally made up of an upper, liquid-permeable cover sheet and a lower, liquid-impermeable cover sheet. An absorption body is arranged between these cover sheets. To prevent urine and excrement leaking out at the sides, i.e. in the transverse direction of the diaper, the latter is normally provided with two side barriers which extend in the longitudinal direction of the diaper, along each side. Moreover, these side barriers are designed so that they are raised slightly from the surface of the diaper. The side barriers can be provided with longitudinal elastic elements, such as threads or bands, in order to give the barriers their desired shape. In addition to having these longitudinal barriers, today's diapers are normally also provided with further longitudinal elements along the side edges, more specifically in the area at the user's crotch. These further longitudinal elements are also made with elastic elements and constitute so-called leg elastic which is intended to ensure that the diaper fits well and provides a seal against the user's legs.

WO 02/49560 discloses an absorbent article comprising side barriers along the longitudinal sides having longitudinal elastic elements which define a folded structure of Z-shaped cross section with a fold directed towards the inside of said article. EP 1 273 281 discloses an absorbent article comprising side barriers along the longitudinal sides having longitudinal elastic elements defining a shape which narrows in the direction towards said front portion. The distance in the transverse direction of the article between said elastic elements is greater at the rear than at the front.

With regard to diapers intended to be used on very small infants or premature babies (who may have a bodyweight of the order of 1 kg), there is a need to abandon the abovementioned known type of diaper construction. In particular, there is a need to use diapers which as far as possible use up a small amount of material, and the material must additionally be especially soft and gentle against the child's skin. For this reason, the abovementioned structure with separate side barriers and leg elastic does not appear to be suitable for diapers intended, for example, for premature babies.

Patent document EP 951890 discloses an absorbent article in the form of a diaper which is of a type comprising two side barriers oriented along a respective side of the diaper. Each of the side barriers comprises two longitudinal elastic elements. This arrangement provides a structure in which each side barrier is raised and additionally forms two longitudinal channels to the inside of each side barrier. The risk of lateral leakage of urine and excrement is reduced in this way. It should be noted that this known diaper has a structure with only one side barrier, in contrast to the type comprising both leg elastic and separate side barriers.

Although the diaper described in EP 951890 affords a basically satisfactory function, there is a need for further improved uptake, distribution and absorption of loose excrement in the type of diapers used on very small infants and in particular in the type used on premature babies. There is a need, not least, to reduce the risk of lateral leakage of loose excrement towards the user's legs. In addition to the need for reliable protection against lateral leakage, there are also corresponding requirements in respect of a reduced risk of excrement leaking out at the rear and up towards the user's back. Moreover, it is particularly required that the type of diaper in question provides a good fit.

### DISCLOSURE OF THE INVENTION

A principal object of the present invention is to make available an improved absorbent article in which the above requirements and needs are satisfied. It is in particular an object of the invention to make available an absorbent article in the form of a diaper, for very small infants and premature babies, which uses a structure with only one side barrier along each side and which effectively prevents leakage of urine and excrement both laterally and at the rear of the article.

The invention is defined by the appended independent claim. Advantageous embodiments are defined by the dependent claims.

The invention affords certain advantages. In particular, it should be noted that the invention provides the possibility of making available an absorbent article such as a diaper which is easy to place on small children, which provides a good fit and which provides good leakage protection both laterally and at the rear of the diaper. The invention also provides a combined function of lateral leakage protection and leg elastic, thereby permitting savings in terms of material and making the article light and flexible for the user. It may be noted in particular that the abovementioned narrowing shape of the elastic elements of the side barriers, in combination with the Z-shaped folded attachment of each side barrier in the front portion of the article, means that each side barrier, during use, is raised very distinctly and effectively in relation to the surface of the diaper. In addition, a cup shape is thus formed which adapts in a reliable manner to the user's anatomy and contributes to the good fit. In addition, a diaper designed according to the invention is simple and inexpensive to produce.

### DESCRIPTION OF THE FIGURES

The invention will be described below with reference to preferred embodiments and to the attached drawings, in which:
- Figure 1: shows a perspective view of an absorbent article in the form of a diaper, in which the present invention can be used,
- Figure 2: shows a diagrammatic and somewhat simplified top view of the diaper according to Figure 1,
- Figure 3: shows a slightly enlarged perspective view of the rear part of the diaper according to Figures 1 and 2, and
- Figure 4: shows a cross-sectional view of the front part of the diaper according to Figures 1 - 3.

### PREFERRED EMBODIMENT

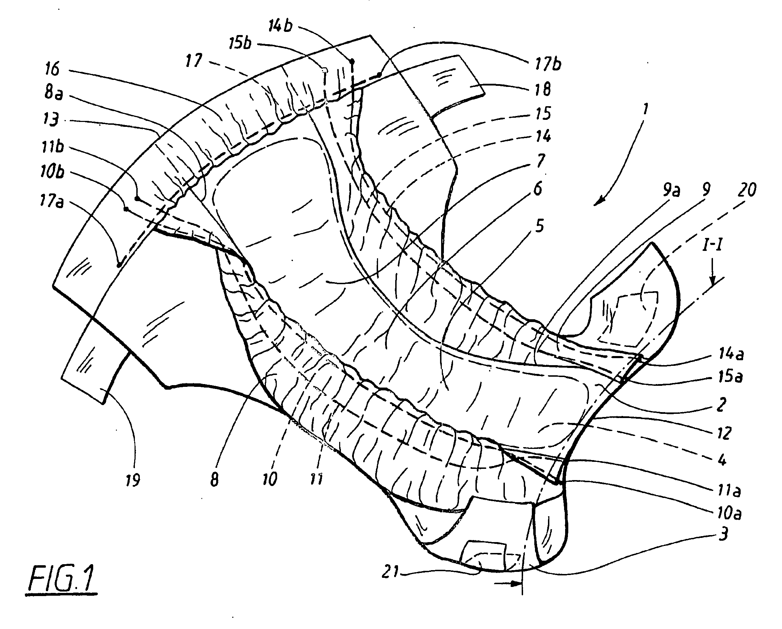

The present invention will now be described with reference to a preferred embodiment. As can be seen from Figure 1, the absorbent article according to the invention is expediently a diaper 1 of the disposable type for infants. The diaper 1 comprises a first cover sheet constituting a liquid-permeable top sheet 2. This top sheet 2 is arranged on that side of the diaper 1 which, during use, is intended to be directed towards the person wearing the diaper. The diaper 1 further comprises a second cover sheet constituting a liquid-tight bottom sheet 3 which, during use, is intended to be directed away from the user, i.e. on the underside of the diaper. An absorption body 4 is arranged between the top sheet 2 and the bottom sheet 3. The absorption body 4 is basically of a type known per se and is designed to rapidly take up liquid excretions on the surface of the diaper 1 and also to convey such excretions to a lower absorbent structure in the absorption body 4. As is already known per se, the absorption body 4 can, for example, comprise a soft wadding material which functions as a layer for conveying and distributing liquid excrement and urine which passes through the top sheet 2. Under this wadding material, the absorption body 4 can expediently comprise a highly absorbent material which optimally takes up and stores bodily excretions. In a possible variant, the absorption body 4 can include what is called a superabsorbent material, for an especially high degree of absorption of liquid. The design of the absorption body 4 in principle follows the known prior art and can include various materials, for example different synthetic and natural fibres, or fibre combinations, and the absorption body 4 can be defined by different material layers or by a single layer with good properties as regards taking up, spreading and storing bodily fluids.

The liquid-permeable top sheet 2 according to Figure 1 expediently consists of a soft nonwoven material, but it can alternatively consist of other materials or material laminates. For example, it can consist of a perforated plastic film, for example of a thermoplastic material such as polyethylene or polypropylene, or a net-like layer of synthetic or textile material. Likewise, different types of laminates of suitable materials can be used as the liquid-permeable upper sheet. The nonwoven materials used are preferably nonwoven fibre layers of natural fibres, for example cellulose fibres or cotton fibres, or synthetic fibres such as polyethylene, polypropylene, polyester, nylon or the like. In addition, mixtures of different fibre types can be used for said nonwoven materials.

The top sheet 2 and the bottom sheet 3 can be joined together (the absorption body 4 being arranged between these layers) with the aid of a suitable joining method, for example adhesive bonding or ultrasonic welding.

Thus, a top sheet 2 made of nonwoven material is preferably used, but, irrespective of the material chosen, the top sheet 2 is intended in a manner known per se to receive and let through liquid excretions from the user and convey these downwards to the underlying absorption body 4. Leakage further through the absorption body 4 is prevented by the underlying liquid-impermeable bottom sheet 3, which is made of a liquid-impermeable material expediently in the form of a thin and liquid-tight plastic film. For example, plastic films made of polyethylene, polypropylene or polyester can be used. A material of the type which is breathable is preferably used for the bottom sheet 3. Moreover, a laminate comprising suitable material layers can be used as the liquid-tight bottom sheet 3.

As can be seen from Figure 1, the diaper 1 has a basically elongated shape and is generally formed to fit around the lower trunk of an infant when in use. For this purpose, the diaper 1 is designed so that it defines, in its longitudinal direction, a front portion 5, a middle portion 6, and a rear portion 7. When the diaper 1 is in use, the front portion 5 is positioned so that it is directed towards the user's belly and down towards the groin area, while the crotch portion 6 is positioned basically directly below the user's crotch, and the rear portion 7 is positioned so that it is directed towards the user's buttocks. The boundaries between the abovementioned portions 5, 6, 7 do not need to be defined with exact dimensions and they do not occur at a specified transverse position for example, but instead along extended transition areas.

As regards the function, dimensions and design of the diaper 1, it is designed in particular to be used on very small infants, including premature babies, in other words babies born before the full term of pregnancy. For this reason, the diaper 1 according to the invention is designed with a special side barrier structure in order to prevent leakage of bodily excretions, in particular in the form of loose and runny excrement, in the transverse direction of the diaper 1, i.e. sideways towards the user's legs. More specifically, the diaper 1 according to the invention is therefore designed with two elongated and longitudinal side edges which form a first elastic side barrier 8 and a second elastic side barrier 9 along the respective side of the diaper 1. As will be described in detail below, these side barriers 8, 9 prevent leakage of urine and excrement out from the diaper towards the user's legs.

The two side barriers 8, 9 are preferably made of a hydrophobic nonwoven material and extend upwards from the respective longitudinal edge of the absorption body 4 so that, when the diaper 1 is in use, they define elastic walls or barriers which bear against and provide a seal against the inside of the user's legs in order to prevent leakage of urine and excrement. In contrast to known diapers which normally comprise separate side barriers and leg elastic components, a fundamental feature of the present invention is that the two side barriers 8, 9 on their own constitute a combined side leakage protection and leg elastic.

The first side barrier 8 is provided with a first elastic element 10 and a second elastic element 11. The first elastic element 10 preferably consists of an elastic thread which is secured near the edge of the first side barrier 8 and runs between two attachment points 10a, 10b on the front edge 12 and rear edge 13, respectively, of the diaper 1. The second elastic element 11 also preferably consists of an elastic thread which extends between the first elastic element 10 and the bottom edge of the side barriers 8 and runs between two attachment points 11a, 11b on the front edge 12 and rear edge 13, respectively of the diaper 1. The two elastic elements 10, 11 are preferably attached at points along the first side barrier 8. However, the invention can also be realized without such attachment, i.e. such that the elastic elements 10, 11 can instead be allowed to run freely along the first side barrier 8.

Correspondingly, the second side barrier 9 is provided with a third elastic element 14 and a fourth elastic element 15. The third elastic element 14 preferably consists of an elastic thread which is arranged near the edge of the second side barrier 9 and runs between two attachment points 14a, 14b on the front edge 12 and rear edge 13, respectively, of the diaper 1. The fourth elastic element 15 also preferably consists of an elastic thread which extends between the third elastic element 14 and the bottom edge of the second side barrier 9 and runs between two attachment points 15a, 15b on the front edge 12 and rear edge 13, respectively, of the diaper 1. In a manner analogous to what has been described above, the third and fourth elastic elements 14, 15 are also preferably attached at points along the second side barrier 9, but alternatively they can also be allowed to run freely along the second side barrier 9. The attachments of the elastic elements 10, 11, 14, 15 are preferably made by adhesive bonding, alternatively by ultrasonic welding. Such attachment methods are already known per se and are therefore not described in detail here.

The two side barriers 8, 9 are secured in the top sheet 2 in a suitable manner, e.g. by ultrasonic welding or adhesive bonding. In this way, a first longitudinal fold 8a is formed where the first side barrier 8 meets the top sheet 2, and a second longitudinal fold 9a is formed where the second side barrier 9 meets the top sheet 2.

In addition to the diaper 1 being intended to provide safety against leakage in the lateral direction, an important aim of the two side barriers 8, 9 is to contribute to the diaper 1 enclosing the user's buttocks in a leaktight manner. This is preferably achieved by virtue of the fact that the side barriers 8, 9 cooperate with a further leakage protection at the waist opening at the rear of the diaper 1. Referring to Figure 1, it will be seen that the diaper 1 according to the invention comprises a rear barrier 16 which runs transverse to the longitudinal direction of the diaper 1, in the area behind the rear portion 7 of the diaper 1. The aim of this rear barrier 16 is mainly to reduce the risk of leakage of loose excrement in the rearward direction, i.e. up towards the user's back. For this purpose, the rear barrier 16 is preferably provided with a fifth elastic element 17 which, like the abovementioned elastic elements 10, 11, 14, 15, preferably consists of an elastic thread running along the edge of the rear barrier 16 in such a way that it extends across all of the abovementioned elastic elements 10, 11, 14, 15. The fifth elastic element 17 ends at two attachment points 17a, 17b, in the manner shown in Figure 1.

In this way, the rear barrier 16 defines a pocket in the rear part of the diaper 1 and cooperates with the two side barriers 8, 9 in such a way that they together form an elastic barrier along the sides and rear part of the diaper 1. The diaper 1 is preferably designed so that the outer attachment points 17a, 17b for the fifth elastic element 17, viewed from above, are positioned outside the rear attachment points 10b, 14b of the outer elastic elements 10, 14.

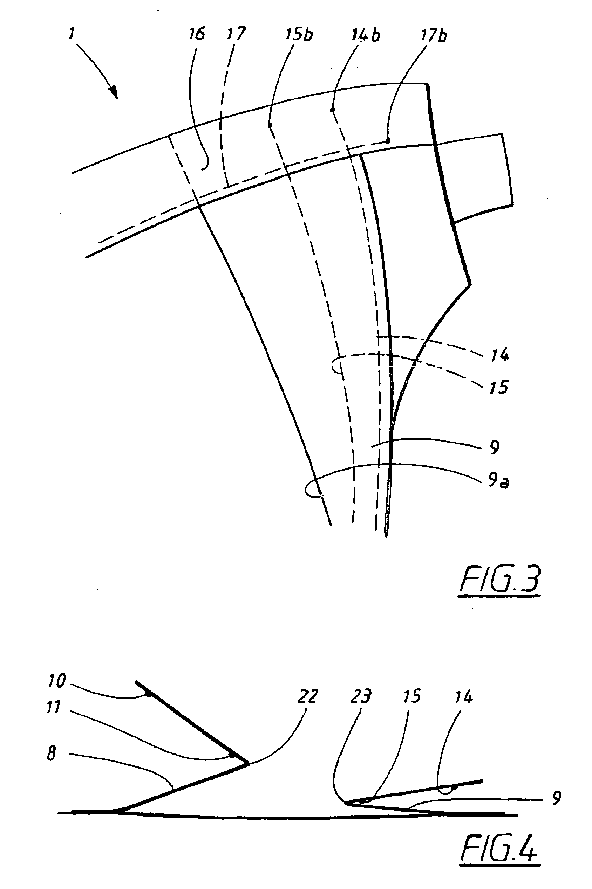

It will further be seen from Figure 1 that each side barrier 8, 9 is arranged in such a way that it is secured in contact with said front portion 5 of the diaper. More precisely, each side barrier 8, 9 is secured in such a way that it defines a structure of substantially Z-shaped cross section, i.e. the side barriers 8, 9 are folded in contact with the front edge and secured in this Z-shaped form. This feature of the invention, which will be described in more detail with reference to Figure 4, helps the diaper 1 open out easily when being used. The rear barrier 16 is also designed to enclose the user's buttocks in an advantageous way between the two side barriers 8, 9 which, at the rear of the diaper 1, are placed at a relatively great distance from each other. In addition, this design of the rear barrier 16 affords a secure seal against leakage of excrement and urine rearwards and over the rear edge of the diaper 1. A further advantage of this design is that the diaper 1 is very easy to fit on the user.

To make the diaper 1 easier to fit on the user, it is designed with two tape-like fastening strips 18, 19 which are arranged in the rear portion 7 of the diaper 1. The fastening strips 18, 19 are intended to cooperate and are secured in a releasable manner against corresponding fastening areas 20, 21 (which are indicated symbolically by broken lines) in the front portion 5 of the diaper 1. The fastening strips 18, 19 can be secured on the fastening areas 20, 21 by means of the fastening strips 18, 19 being provided with a suitable adhesive for example, or alternatively by their being designed as velcro-type fasteners or in some other suitable manner. As an alternative to the fastening areas 20, 21 shown in Figure 1, the diaper 1 can be provided with a single fastening area which can then be located centrally on the front portion of the diaper. This contributes to an improved fit of the diaper 1.

In Figure 1, the diaper 1 according to the invention is shown as it appears during use. The figure shows that each side barrier 8, 9 is raised relatively high from the top sheet 2 of the diaper 1. It should also be noted that the diaper 1 does not comprise any separate leg elastic as in conventional diapers. The design according to the invention affords advantages, especially in the form of limited material consumption, a better fit, and a reduced risk of leakage over the sides. The rear elastic barrier 16 also provides a greater possibility of taking up loose excrement in the rearward direction, i.e. towards the user's back.

The requirement relating to effective sealing and safety against leakage in the rearward direction over the opening defined at the waist is satisfied in particular by the rear elastic barrier 16 and its interaction with the two side barriers 8, 9. Since the distance between the two side barriers 8, 9 at the rear of the diaper 1 is relatively large, effective sealing is required at the waist opening in order to prevent leakage. According to the invention, this is achieved in particular by the fact that the rear barrier 16 comprises the abovementioned fifth elastic element 17 (expediently consisting of an elastic thread) which preferably extends at least as far as the outermost elastic elements of the side barriers 8, 9, i.e. the abovementioned first elastic element 10 and fourth elastic element 14. More precisely, attachment points for the fifth elastic element 17 are chosen which provide a continuous barrier along the longitudinal sides and the waist opening at the rear.

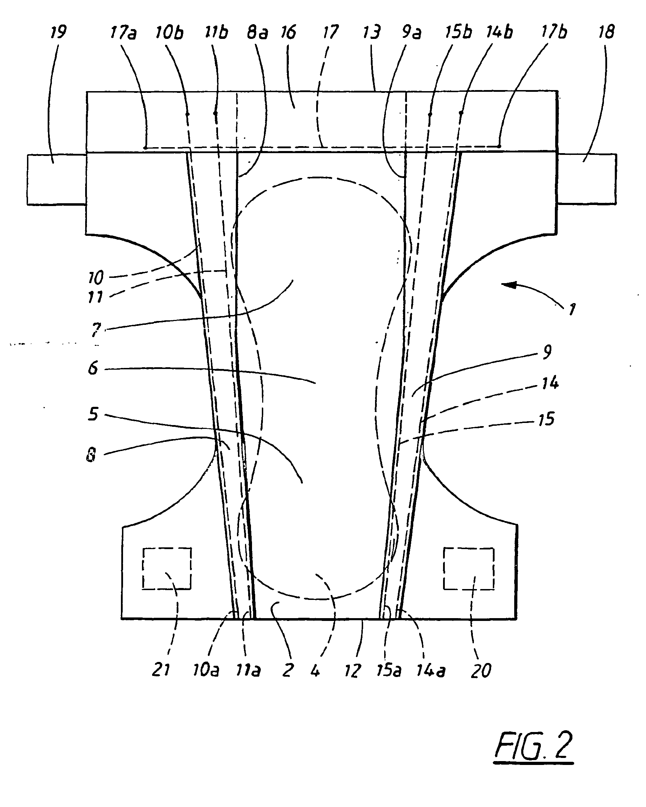

As can be seen from Figure 2, which is a diagrammatic top view of the diaper 1 according to the invention, another fundamental feature of the invention is that the distance between the rear attachment point 10b of the first elastic element 10 and the rear attachment point 14b of the third elastic element 14 is greater than the distance between the front attachment point 10a of the first elastic element 10 and the front attachment point 14a of the third elastic element 14. Correspondingly, the distance between the rear attachment point 11 b of the second elastic element 11 and the rear attachment point 15b of the fourth elastic element 15 is greater than the distance between the front attachment point 11 a of the second elastic element 11 and the front attachment point 15a of the fourth elastic element 15. This means that the four elastic elements 10, 11, 14, 15, viewed from above, run alongside each other in such a way that, together with the respective side barrier 8, 9, they form a narrowing structure, i.e. in the direction from the rear portion 7 of the diaper 1 towards its front portion 5. By virtue of the fact that the distance of the side barriers 8, 9 from each other is therefore less at the front than at the rear of the diaper 1, the latter is easy to fit on the user.

It should be noted in particular that the rear attachment point 10b of the first elastic element 10 lies outside the rear attachment point 11 b of the second elastic element 11 (viewed in relation to an imaginary longitudinal axis of symmetry running along the longitudinal direction of the diaper 1). Likewise, the rear attachment point 14b of the third elastic element 14 lies outside the rear attachment point 15b of the fourth elastic element 15.

The attachment of the elastic elements 10, 11, 14, 15 in the front portion 5 of the diaper 1 is analogous to the attachment in the rear portion 7. Thus, the front attachment point 10a of the first elastic element 10 lies outside the front attachment point 11 a of the second elastic element 11, while the front attachment point 14a of the third elastic element 14 lies outside the front attachment point 15a of the fourth elastic element 15. However, it should be noted that the actual material of the respective side barrier 8, 9 is folded like a Z at the attachment to the front portion of the diaper 1, as has been described above.

As can be seen from Figures 1 and 2, the rear part of each side barrier 8, 9 is designed so that it forms an outwardly folded and essentially open, cup-shaped structure by virtue of the fact that the first elastic element 10 is attached to the rear of the diaper 1 at a point 10b which, viewed from above, lies outside the attachment point 11 b for the second elastic element 11 and the first longitudinal fold 8a. Correspondingly, the attachment point 14b for the third elastic element 14 lies outside the attachment point 15b for the fourth elastic element 15 and outside the second longitudinal fold 9a. In this way, the side barriers 8, 9 will lift effectively and form a cup-like structure during use. This is facilitated by the fact that the respective side barrier 8, 9 additionally comprises two longitudinal elastic elements each (10, 11 and 14, 15, respectively). The narrowing geometry in the forward direction defined by the side barriers 8, 9 and the elastic elements 10, 11, 14, 15, and the Z-shaped attachment at the front, also contribute to stretching and lifting the side barriers 8, 9.

To ensure that the side barriers 8, 9 will enclose the user's buttocks and will not get in the way when placing the front part of the diaper 1 on the user, the distance between the rear attachment points 10b, 14b of the laterally outer elastic elements 10, 14 (outer edges of the barrier) should be at least twice as great, preferably at least three times as great, as the corresponding distance at the front, i.e. the distance between the front attachment point 10a of the first elastic element 10 and the front attachment point 14a of the third elastic element 14.

Moreover, the distance between the front attachment point 11a of the second elastic element 11 and the front attachment point 15a of the fourth elastic element 15 is preferably within the range of 1 - 3 cm. In this way, a space is created, at the front of the diaper 1, which can be considered sufficiently large for small infants, but which is not so wide as to prevent application of the diaper 1.

Figure 3 shows a perspective view of the rear part of the diaper 1 according to Figures 1 and 2. More precisely, Figure 3 shows in detail how the rear part of the second side barrier 9 is attached in the rear part of the diaper 1. This attachment allows the side barrier 9 to be folded out so that a cup-shaped structure is formed. This is achieved by the fact that the third elastic element 14 runs along the edge of the second side barrier 9 and onwards under the rear barrier 16 to its rear attachment point 14b. This rear attachment point 14b is thus situated inside the second side barrier 9, which in turn extends under the rear barrier 16. The fourth elastic element 15 also runs along the second side barrier 9 and onwards under the rear barrier 16 to its rear attachment point 15b. The second longitudinal fold 9a then runs along the distance where the second side barrier 9 meets the top sheet 2 and ends under the rear barrier 16. The fifth elastic element 17 runs across the rear section of the second side barrier 16 so that a rear pocket is formed, the fifth elastic element 17 having one attachment point 17a outside the third elastic element 14 and the fourth elastic element 15 (viewed from above).

The Z-folded structure at the front part of each side barrier 8, 9 can be seen clearly from Figure 4, which is a somewhat simplified and diagrammatic view of a cross section through the diaper 1 according to the invention, viewed at the front edge of the diaper 1, as is indicated by the line I-I in Figure 1. The left-hand part of Figure 4 shows an imaginary situation during production and shaping of the structure to give the Z-folded configuration. The right-hand part of Figure 4 shows the structure after it has been folded. Figure 4 shows how the first side barrier 8 is folded and also supports the first elastic element 10 and the second elastic element 11. Correspondingly, the second side barrier 9 is folded and supports the third elastic element 14 and the fourth elastic element 15. Two folds 22, 23 are thus formed in the respective side barrier 8, 9, namely a first fold 22 in the first side barrier 8 and a second fold 23 in the second side barrier 9. These folds 22, 23 are directed towards the inside of the diaper 1, i.e. in the direction towards an imaginary longitudinal axis of symmetry of the diaper 1. The inner elastic elements 11, 15, i.e. the second elastic element 11 and the fourth elastic element 15, are thus positioned at or near the respective fold 22, 23 which is formed. The other elastic threads 10, 14 are then applied where suitable between the inner threads 11, 15 and the outermost edge of the respective side barrier 8, 9.

It is therefore a principle of the invention that the side barriers 8, 9 are arranged such that their respective front portion is folded in a Z shape, the front part of the second elastic element 11 and the fourth elastic element 15 being positioned in contact with the inner folds 22, 23. The rear part of the respective side barrier 8, 9, however, defines no such Z-shaped fold but instead forms raised side walls which contribute to the cup-shaped and leaktight rear part 7 of the diaper 1. The fact that the side barriers 8, 9 are folded out in the rear portion 7 of the diaper 1 and are folded in a Z shape in the front portion 5 of the diaper 1 ensures a good fit of the diaper 1. The diaper 1 is also made easy to place on a user and permits effective lifting of the side barriers 8, 9 during use of the diaper 1, a good fit and a high degree of protection against leaking.

Each side barrier 8, 9 is thus attached with the Z-shaped configuration at the front end of the respective side barrier 8, 9 (see Figure 1). The Z-folded portion expediently extends from the front edge 12 of the diaper and at least as far as the front edge of the absorption body 4. However, the invention is not limited to this, and instead the Z-folded portion can alternatively have another extent.

The invention is not limited to the embodiments described above and instead it can be varied within the scope of the attached claims. For example, different types of elastic elements can be used in the respective side barrier 8, 9, for example threads, bands, films, nonwoven material or the like. The number of elastic elements can also be varied, although, if they are in the form of threads, they must be at least two in number. If threads are used, two or more threads can be used in the side barriers 8, 9 so as to distribute the pressure of the elastic against the sensitive body of the child.

If, for example, a wide elastic is used, this should extend from the respective fold in the Z shape, in the same way as the innermost elastic thread must be applied at or near the fold (see Figure 3).

Although this has not been described above, a diaper according to an alternative embodiment can have further elastic elements, preferably in the form of two coil-like portions, along the front end edge 12 and the rear end edge 13. These elastic elements can be formed to close against the user's stomach and back, respectively, by which means the diaper acquires the desired fit and comfort during use.

## Claims

1. Absorbent article (1) comprising an upper, liquid-permeable cover sheet (2), a lower, liquid-impermeable cover sheet (3), and an absorption body (4) arranged between the cover sheets (2, 3), said article (1) defining a longitudinal direction, a front portion (5) in the longitudinal direction, a rear portion (7), and a middle portion (6) arranged between said portions (5, 7), said article (1) additionally comprising first and second side barriers (8, 9) along the respective longitudinal side, and each side barrier (8, 9) in turn comprising at least one longitudinal elastic element (10, 11; 14, 15), said first and second side barriers (8, 9), viewed from above, defining a shape which narrows in the direction towards said front portion (5) so that the distance, in the transverse direction of the article (1), between said elastic elements (10, 11, 14, 15) is greater in said rear portion (7) than in said front portion (5), wherein each side barrier (8, 9) is arranged such that, when it is secured in contact with said front portion (5), it defines a folded structure of substantially Z-shaped cross section with a fold (22, 23) directed towards the inside of said article (1); **characterized in that** the absorbent article (1) comprises a rear barrier (16) formed in said rear portion (7), which rear barrier (16) is intended for taking up bodily excretions in the direction rearwards along said article (1); said rear barrier (16) comprises a further elastic element (17); and said further elastic element (17) comprises attachment points which, viewed from above, extend outside and overlap rear attachment points (10b, 14b) of the elastic elements (10, 14) in said side barriers (8, 9), a barrier being defined along the longitudinal sides and rear side of said article (1).

2. Absorbent article (1) according to claim 1, **characterized in that** the first side barrier (8) is secured in said cover sheet (2) so that a first longitudinal fold (8a) is defined, and **in that** the second side barrier (9) is secured in said cover sheet (2) so that a second longitudinal fold (9a) is defined.

3. Absorbent article (1) according to claim 2, **characterized in that** the elastic element (10, 11) in the first side barrier (8), viewed from above, is secured in said rear portion (7) outside the first longitudinal fold (8a), and **in that** the elastic element (14, 115) in the second side barrier (9), viewed from above, is secured in said rear portion (7) outside the second longitudinal fold (9a).

4. Absorbent article (1) according to any of the preceding claims, **characterized in that** said first side barrier (8) comprises a first elastic element (10) and a second elastic element (11), **in that** said second side barrier (9) comprises a third elastic element (14) and a fourth elastic element (15), said first elastic element (10) extending outside the second elastic element (11) viewed in relation to a longitudinal axis of symmetry through said article (1), and said third elastic element (14) extending outside the fourth elastic element (15) viewed in relation to said axis of symmetry.

5. Absorbent article (1) according to any of the preceding claims, **characterized in that** the distance between the elastic elements (10,14) of the side barriers (8, 9) is at least two times, preferably three times, greater at said rear portion (7) than at said front portion (5).

6. Absorbent article (1) according to any of the preceding claims, **characterized in that** the distance between those parts of the elastic elements (11a, 15a) of the side barriers (8, 9) nearest to the inside of said article (1) is within the range of 1 - 3 cm at said front portion (5).

7. Absorbent article (1) according to any of the preceding claims, **characterized in that** said elastic elements (11, 15) run at least partially in contact with said folds (22, 23) in each side barrier (8, 9).

8. Absorbent article (1) according to any of the preceding claims, **characterized in that** said first and second side barriers (8, 9) by themselves constitute a combined side leakage protection and leg elastic for said article (1).

9. Absorbent article (1) according to any of the preceding claims, **characterized in that** said elastic elements (10, 11, 14, 15) consist of elastic threads.

## Patentansprüche

1. Absorbierender Artikel (1), umfassend eine obere, flüssigkeitsdurchlässige Abdeckschicht (2), eine untere, flüssigkeitsundurchlässige Abdeckschicht (3) und einen absorbierenden Körper (4), der zwischen den Abdeckschichten (2, 3) angeordnet ist, wobei der Artikel (1) eine Längsrichtung, einen vorderen Teil (5) in der Längsrichtung, einen hinteren Teil (7) und einen mittleren Teil (6), der zwischen den Teilen (5, 7) angeordnet ist, definiert, wobei der Artikel (1) darüber hinaus eine erste und eine zweite Seitensperre (8, 9) entlang der jeweiligen Längsseite umfasst, und jede Seitensperre (8, 9) wiederum mindestens ein elastisches Längselement (10, 11; 14, 15) umfasst, wobei die erste und die zweite Seitensperre (8, 9), von oben gesehen, eine Form definieren, die sich in auf den vorderen Teil (5) verlaufender Richtung verschmälert, so dass der Abstand in Querrichtung des Artikels (1) zwischen den elastischen Elementen (10, 11, 14, 15) in dem hinteren Teil (7) größer ist als in dem vorderen Teil (5), wobei jede Seitensperre (8, 9) so angeordnet ist, dass sie, wenn sie in Kontakt mit dem vorderen Teil (5) befestigt ist, eine gefaltete Struktur mit im Wesentlichen Z-förmigem Querschnitt mit einer zur Innenseite des Artikels (1) gerichteten Falte (22, 23) definiert;
**dadurch gekennzeichnet, dass** der absorbierende Artikel (1) eine hintere Sperre (16) umfasst, die in dem hinteren Teil (7) ausgebildet ist, wobei die hintere Sperre (16) Körperausscheidungen in entlang dem Artikel (1) nach hinten verlaufender Richtung aufnehmen soll; wobei die hintere Sperre (16) ein weiteres elastisches Element (17) umfasst; und das weitere elastische Element (17) Befestigungsstellen umfasst, die sich, von oben gesehen, außerhalb hinterer Befestigungsstellen (10b, 14b) der elastischen Elemente (10, 14) in den Seitensperren (8, 9) erstrecken und diese überlappen, wobei eine Sperre entlang den Längsseiten und der Rückseite des Artikels (1) definiert wird.

2. Absorbierender Artikel (1) nach Anspruch 1, **dadurch gekennzeichnet, dass** die erste Seitensperre (8) so in der Abdeckschicht (2) befestigt ist, dass eine erste Längsfalte (8a) definiert wird, und dass die zweite Seitensperre (9) so in der Abdeckschicht (2) befestigt ist, dass eine zweite Längsfalte (9a) definiert wird.

3. Absorbierender Artikel (1) nach Anspruch 2, **dadurch gekennzeichnet, dass** das elastische Element (10, 11) in der ersten Seitensperre (8), von oben gesehen, in dem hinteren Teil (7) außerhalb der ersten Längsfalte (8a) befestigt ist, und dass das elastische Element (14, 115) in der zweiten Seitensperre (9), von oben gesehen, in dem hinteren Teil (7) außerhalb der zweiten Längsfalte (9a) befestigt ist.

4. Absorbierender Artikel (1) nach einem der vorhergehenden Ansprüche, **dadurch gekennzeichnet, dass** die erste Seitensperre (8) ein erstes elastisches Element (10) und ein zweites elastisches Element (11) umfasst, dass die zweite Seitensperre (9) ein drittes elastisches Element (14) und ein viertes elastisches Element (15) umfasst, wobei sich das erste elastische Element (10) außerhalb des zweiten elastischen Elements (11), mit Blickrichtung bezüglich einer Längssymmetrieachse durch den Artikel (1), erstreckt und sich das dritte elastische Element (14) außerhalb des vierten elastischen Elements (15), mit Blickrichtung bezüglich der Symmetrieachse, erstreckt.

5. Absorbierender Artikel (1) nach einem der vorhergehenden Ansprüche, **dadurch gekennzeichnet, dass** der Abstand zwischen den elastischen Elementen (10, 14) der Seitensperren (8, 9) an dem hinteren Teil (7) mindestens zweimal, vorzugsweise dreimal so groß ist wie an dem vorderen Teil (5).

6. Absorbierender Artikel (1) nach einem der vorhergehenden Ansprüche, **dadurch gekennzeichnet, dass** der Abstand zwischen diesen Teilen der elastischen Elemente (11a, 15a) der Seitensperren (8, 9) am nächsten zu der Innenseite des Artikels (1) am vorderen Teil (5) in einem Bereich von 1 -3 cm liegt.

7. Absorbierender Artikel (1) nach einem der vorhergehenden Ansprüche, **dadurch gekennzeichnet, dass** sich die elastischen Elemente (11, 15) zumindest teilweise in Kontakt mit den Falten (22, 23) in jeder Seitensperre (8, 9) verlaufen.

8. Absorbierender Artikel (1) nach einem der vorhergehenden Ansprüche, **dadurch gekennzeichnet, dass** die erste und zweite Seitensperre (8, 9) alleine einen kombinierten Seitenleckschutz und einen elastischen Beinabschluss für den Artikel (1) bilden.

9. Absorbierender Artikel (1) nach einem der vorhergehenden Ansprüche, **dadurch gekennzeichnet, dass** die elastischen Elemente (10, 11, 14, 15) aus elastischen Fäden bestehen.

## Revendications

1. Article absorbant (1) comprenant une feuille de revêtement supérieure (2) perméable aux liquides, une feuille de revêtement inférieure (3) imperméable aux liquides, et un corps absorbant (4) agencé entre les feuilles de revêtement (2, 3), ledit article (1) définissant une direction longitudinale, une portion avant (5) dans la direction longitudinale, une portion arrière (7), et une portion centrale (6) agencée entre lesdites portions (5, 7), ledit article (1) comprenant en outre des première et deuxième barrières latérales (8, 9) le long du côté longitudinal respectif, et chaque barrière latérale (8, 9), à son tour, comprenant au moins un élément élastique longitudinal (10, 11; 14, 15), lesdites première et deuxième barrières latérales (8, 9), vues de dessus, définissant une forme qui rétrécit dans la direction de ladite portion avant (5) de telle sorte que la distance, dans la direction transversale de l'article (1), entre lesdits éléments élastiques (10, 11, 14, 15) soit plus grande dans ladite portion arrière (7) que dans ladite portion avant (5), chaque barrière latérale (8, 9) étant agencée de telle sorte que, lorsqu'elle est fixée en contact avec ladite portion avant (5), elle définisse une structure pliée de section transversale sensiblement en forme de Z avec un pli (22, 23) orienté vers l'intérieur dudit article (1); **caractérisé en ce que** l'article absorbant (1) comprend une barrière arrière (16) formée dans ladite portion arrière (7), laquelle barrière arrière (16) est destinée à recevoir les excrétions corporelles dans la direction de l'arrière le long dudit article (1); ladite barrière arrière (16) comprend un élément élastique supplémentaire (17); et que ledit élément élastique supplémentaire (17) comprend des points d'attache qui, vus de dessus, s'étendent à l'extérieur et chevauchent des points d'attache arrière (10b, 14b) des éléments élastiques (10, 14) dans lesdites barrières latérales (8, 9), une barrière étant définie le long des côtés longitudinaux et du côté arrière dudit article (1).

2. Article absorbant (1) selon la revendication 1, **caractérisé en ce que** la première barrière latérale (8) est fixée dans ladite feuille de revêtement (2) de telle sorte qu'un premier pli longitudinal (8a) soit défini, et **en ce que** la deuxième barrière latérale (9) est fixée dans ladite feuille de revêtement (2) de telle sorte qu'un deuxième pli longitudinal (9a) soit défini.

3. Article absorbant (1) selon la revendication 2, **caractérisé en ce que** l'élément élastique (10, 11) dans la première barrière latérale (8), vu de dessus, est fixé dans ladite portion arrière (7) à l'extérieur du premier pli longitudinal (8a), et **en ce que** l'élément élastique (14, 115) dans la deuxième barrière latérale (9), vu de dessus, est fixé dans ladite portion arrière (7) à l'extérieur du deuxième pli longitudinal (9a).

4. Article absorbant (1) selon l'une quelconque des revendications précédentes, **caractérisé en ce que** ladite première barrière latérale (8) comprend un premier élément élastique (10) et un deuxième élément élastique (11), **en ce que** ladite deuxième barrière latérale (9) comprend un troisième élément élastique (14) et un quatrième élément élastique (15), ledit premier élément élastique (10) s'étendant à l'extérieur du deuxième élément élastique (11), vu par rapport à un axe de symétrie longitudinal à travers ledit article (1), et ledit troisième élément élastique (14) s'étendant à l'extérieur du quatrième élément élastique (15), vu par rapport audit axe de symétrie.

5. Article absorbant (1) selon l'une quelconque des revendications précédentes, **caractérisé en ce que** la distance entre les éléments élastiques (10, 14) des barrières latérales (8, 9) est au moins deux fois, de préférence trois fois, plus grande au niveau de ladite portion arrière (7) qu'au niveau de ladite portion avant (5).

6. Article absorbant (1) selon l'une quelconque des revendications précédentes, **caractérisé en ce que** la distance entre les parties des éléments élastiques (11a, 15a) des barrières latérales (8, 9) les plus proches de l'intérieur dudit article (1) est comprise dans la plage de 1 à 3 cm au niveau de ladite portion avant (5).

7. Article absorbant (1) selon l'une quelconque des revendications précédentes, **caractérisé en ce que** lesdits éléments élastiques (11, 15) s'étendent au moins en partie en contact avec lesdits plis (22, 23) dans chaque barrière latérale (8, 9).

8. Article absorbant (1) selon l'une quelconque des revendications précédentes, **caractérisé en ce que** lesdites première et deuxième barrières latérales (8, 9) constituent elles-mêmes une protection combinée contre les fuites latérales et un élastique de jambe pour ledit article (1).

9. Article absorbant (1) selon l'une quelconque des revendications précédentes, **caractérisé en ce que** lesdits éléments élastiques (10, 11, 14, 15) sont constitués de fils élastiques.
